Europäisches Patentamt

European Patent Office

Office européen des brevets

(19)

(11) Veröffentlichungsnummer: **0 379 216 B1**

(12) **EUROPÄISCHE PATENTSCHRIFT**

(45) Veröffentlichungstag der Patentschrift: **08.06.94**

(21) Anmeldenummer: **90101095.9**

(22) Anmeldetag: **19.01.90**

Die Akte enthält technische Angaben, die nach dem Eingang der Anmeldung eingereicht wurden und die nicht in dieser Patentschrift enthalten sind.

(51) Int. Cl.5: **G01N 33/531**, G01N 33/58, G01N 33/569, G01N 33/532

(54) **Verfahren und Reagenz zur Bestimmung von immunologisch nachweisbaren Substanzen.**

(30) Priorität: **20.01.89 DE 3901638**
**21.07.89 DE 3924239**

(43) Veröffentlichungstag der Anmeldung:
**25.07.90 Patentblatt 90/30**

(45) Bekanntmachung des Hinweises auf die Patenterteilung:
**08.06.94 Patentblatt 94/23**

(84) Benannte Vertragsstaaten:
**AT BE CH DE DK ES FR GB GR IT LI LU NL SE**

(56) Entgegenhaltungen:
**EP-A- 0 005 271**
**EP-A- 0 173 295**
**FR-A- 2 601 455**
**GB-A- 2 181 840**

(73) Patentinhaber: **BOEHRINGER MANNHEIM GMBH**

**D-68298 Mannheim(DE)**

(72) Erfinder: **Bayer, Hubert, Dr. rer. nat.**
**Am Ölschlag 2**
**D-8120 Weilheim(DE)**

Erfinder: **Kirch, Peter, Dr. rer. nat.**
**Weilheimer Strasse 26**
**D-8911 Rott(DE)**
Erfinder: **Kopetzki, Erhard, Dr. rer. nat.**
**Henlestrasse 2**
**D-89122 Penzberg(DE)**
Erfinder: **Klein, Christian, Dr. rer. nat.**
**Blütenstrasse 16**
**D-8120 Weilheim(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**Beschreibung**

Die Erfindung betrifft ein Verfahren zur Bestimmung von immunologisch nachweisbaren Substanzen nach dem Prinzip des heterogenen Immunoassays durch Inkubation mit mindestens zwei Rezeptoren $R_1$ und $R_2$, von denen $R_1$ die Bindung an die feste Phase vermittelt und $R_2$ ein Konjugat ist aus einem mit einer zu bestimmenden Substanz bindefähigen Liganden und einer Markierung unter Verwendung einer Festphase, an die eine spezifisch bindefähige Komponente gebunden ist, Trennung der festen von der flüssigen Phase und Bestimmung der Markierung in einer der beiden Phasen sowie ein dazu geeignetes Reagenz.

Eine Vielzahl klinisch wertvoller Parameter wird mit immunologischen Nachweisverfahren bestimmt. Für die Immunoassays gibt es eine große Auswahl von homogenen und heterogenen Verfahrensvarianten. Häufig wird ein Verfahren nach dem Sandwich-Prinzip oder einer davon abgeleiteten Verfahrensvariante benutzt. Üblicherweise wird die Probelösung mit einem Rezeptor, der mit der nachzuweisenden Substanz bindefähig ist und eine Markierung trägt und einem an die Festphase gebundenen, oder einem die Bindung an die feste Phase vermittelnden, mit der nachzuweisenden Substanz spezifisch bindefähigen Rezeptor inkubiert. Dabei bilden sich an die Festphase über den die Bindung an die Festphase vermittelnden Rezeptor gebundene Komplexe von nachzuweisender Substanz und markiertem Rezeptor. Durch Trennung der festen von der flüssigen Phase kann die gebundene Markierung von der ungebundenen Markierung leicht isoliert werden und in einer der beiden Phasen nachgewiesen werden. Die Menge der Markierung ist ein Maß für den Gehalt an nachzuweisender Substanz.

Es gibt nun verschiedene Situationen, bei denen nicht nur ein Test, sondern parallel mehrere verschiedene Tests durchgeführt werden müssen. Dies ist beispielsweise der Fall bei der Diagnostik von gegen bestimmte Viren gerichteten Antikörpern, wie z.B. Anti-HIV-Antikörpern oder gegen Hepatitis-Antigene gerichteten Antikörpern. Generell werden gegen ein Antigen oder Hapten vom Körper eine Vielzahl verschiedener Antikörper gebildet, d.h. Antikörper, die mit verschiedenen Epitopen bindefähig sind. Einige Viren haben die Eigenschaft, ihre Oberfläche ständig zu verändern, so daß es sehr schwierig ist, entweder Antikörper zu finden, die einen zuverlässigen Nachweis des Antigens erlauben oder Modellsubstanzen zu finden, mit denen alle gegen ein bestimmtes Virus gebildeten Antikörper erfaßt werden können. Aus diesem Grunde versucht man in parallelen Tests durch Einsatz verschiedener Antikörper, die mit verschiedenen viralen Antigenen bindefähig sind, oder durch Einsatz verschiedener antigener Determinanten, die Nachweisgenauigkeit zu erhöhen. Weiterhin ist es bei vielen Krankheitsbildern wichtig, parallel verschiedene Parameter nachzuweisen, beispielsweise in der Tumordiagnostik bei der Suche nach Tumormarkern oder bei der Untersuchung von Allergie-Patienten für den Nachweis von Allergenen bzw. von Anti-Allergen-Antikörpern. Auch bei der Untersuchung von Blut für Transfusionen ist es notwendig, verschiedene Tests parallel auszuführen, um festzustellen, ob das Blut irgendwelche Risikofaktoren wie HIV-Antikörper, Hepatitis-Erreger etc. enthält.

Bisher ist es sehr aufwendig, verschiedene Antigene oder Antikörper parallel nachzuweisen, da für jede einzelne Substanz ein gesonderter Test durchgeführt werden muß, was zeit- und arbeitsaufwendig ist. Es wurde auch schon vorgeschlagen, simultan mehrere Antikörper nebeneinander zu bestimmen, indem man verschiedene Antigene an der Festphase immobilisiert (vgl. z.B. W0/89/01527). Dieses Verfahren brachte jedoch in der Regel keine befriedigenden Ergebnisse, da einerseits Störreaktionen auftraten und andererseits für solche Tests die Bindekapazität der Festphase so gering war, daß die Empfindlichkeit unter den Anforderungen der Praxis lag.

EP-A-0 173 295 beschreibt ein Verfahren zum Nachweis des Vorhandenseins eines ersten Antigens eines ersten Pathogens oder eines ersten Antikörpers eines zweiten Pathogens in einer Testprobe umfassend: (a) Leiten der Testprobe über einen festen Träger, an dem ein Antikörper gegen das erste Antigen und ein Antigen gegen den ersten Antikörper befestigt sind, (b) Leiten eines zweiten Antikörpers gegen das erste Antigen, wobei der zweite Antikörper von einer unterschiedlichen Tierspezies ist, und eines Antikörpers gegen den ersten Antikörper von einer unterschiedlichen Tierspezies über die resultierende Zusammensetzung von Schritt (a), (c) immunologisches Nachweisen des Vorhandenseins des zweiten Antikörpers gegen das erste Antigen und des Antikörpers gegen den ersten Antikörper. Bei diesem Verfahren kann jedoch keine Unterscheidung durchgeführt werden, welche der zu bestimmenden Substanzen tatsächlich vorhanden ist, so daß die Identifikation eines speziellen Pathogens, Antigens, Antikörpers oder Enzymmarkers oder einer Kombination davon durch erneutes Testen auf jede einzelne Substanz nach üblichen Methoden erfolgen muß.

Es war nun Aufgabe der vorliegenden Erfindung, ein Verfahren zur Verfügung zu stellen, mit dem mehrere Parameter gleichzeitig mit hoher Genauigkeit und geringem Zeitaufwand bestimmt werden können.

Diese Aufgabe wird gelöst durch ein Verfahren zur Bestimmung von zwei oder mehr immunologisch nachweisbaren Substanzen nach dem Prinzip des heterogenen Immunoassays, bei dem man über eines der Bindepaare Biotin/Avidin oder Biotin/Streptavidin mehrere verschiedene Rezeptoren $R_1$, welche Bindestellen für eine der zu bestimmenden Substanzen aufweisen, an eine feste Phase koppelt, wobei die Kopplung so erfolgt, daß einer der beiden Partner des Bindesystems an ein lösliches Protein mit einem Molekulargewicht über 500000 gebunden wird, das dann an der festen Phase adsorbiert wird, und der andere Partner des Bindesystems an die Rezeptoren $R_1$ gebunden wird, die feste Phase mit den daran gebundenen Rezeptoren $R_1$ mit einer Probelösung in Kontakt bringt, welche die zu bestimmenden Substanzen enthält, und mit Hilfe von Rezeptoren $R_2$, welche Konjugate aus einem mit einer zu bestimmenden Substanz bindefähigen Liganden una einer Markierung sind, die An- oder Abwesenheit der Substanzen nachweist.

Überraschenderweise gelingt es mit dem erfindungsgemäßen Verfahren, simultan verschiedene Substanzen zu bestimmen. Es kann in einfacher Weise und mit geringem Zeitaufwand ein ganzes Spektrum gewünschter Parameter abgedeckt werden.

Das erfindungsgemäße Verfahren eignet sich für alle Varianten des heterogenen Immunoassays.

Für das erfindungsgemäße Verfahren wird eine feste Phase verwendet, an deren Oberfläche spezifisch bindende Partner gebunden sind. Für die Festphase können die an sich bekannten Materialien wie Kunststoff, Glas, Papierträger, Keramik, Latex, Magnetteilchen und andere verwendet werden. Die feste Phase kann z.B. in Form von Reaktionsröhrchen, Reagenzträgerstreifen oder Kugeln vorliegen. In einer bevorzugten Ausführungsform liegt die feste Phase in Form eines Reaktionsgefäßes, besonders bevorzugt als Küvette ausgebildet vor, dessen Wände zumindest teilweise an der Innenoberfläche mit spezifisch bindenden Partnern beschichtet sind. Als Material für das Reaktionsgefäß sind die bekannten Materialien geeignet. Bevorzugt sind hier Polystyrol, Copolymere des Polystyrols, Polycarbonate, Polyacrylate und Polymethacrylate.

Die Beschichtung des Trägermaterials mit den spezifisch bindenden Partnern kann durch die Bindung an ein lösliches Protein mit einem Molekulargewicht über 500.000, das dann an der Innenoberfläche des Reaktionsgefäßes adsorbiert wird, erfolgen.

Verfahren und Mittel hierzu sind bekannt. In einer bevorzugten Ausführungsform wird als feste Phase ein Trägermaterial verwendet, das nach dem in DE-A-36 40 412.8 beschriebenen Verfahren hergestellt wurde. In einer weiteren bevorzugten Ausführungsform ist die feste Phase ein Reagenzträger, der erhalten wurde, indem ein Faservlies aus einer Cellulose/Synthesefasermischung durch Behandlung mit Perjodat aktiviert wurde und mit dem spezifisch bindenden Partner, der vorher sauer behandelt wurde, beladen wurde. Ein Verfahren zur Herstellung derartiger Reagenzträger ist beschrieben in DE-A-35 43 749.

An die Oberfläche der Festphase wird ein spezifisch bindender Partner gebunden. Dieser Partner muß mit einem Teil von Rezeptor $R_1$ bindefähig sein. Da die Bindung zwischen dem an der Festphase gebundenen Partner und dem Rezeptor $R_1$ die Bindefähigkeit des Rezeptors $R_1$ mit der nachzuweisenden Substanz nicht beeinträchtigen soll, werden entweder, wenn Rezeptor $R_1$ ein Antikörper ist, gegen den Fc-Teil dieses Antikörpers gerichtete Antikörper oder Protein A verwendet oder es werden Partner P1,P2 eines spezifisch bindenden Paares immobilisiert. Als spezifisch bindende Paare sind Biotin-Streptavidin sowie Biotin-Avidin geeignet. Bevorzugt werden an der Festphase mit Biotin bindefähige Partner immobilisiert, insbesondere Streptavidin oder Avidin.

Die Probelösung wird mit verschiedenen Rezeptoren $R_1$ inkubiert. Als Rezeptoren $R_1$ werden Rezeptoren verwendet, die sowohl Bindestellen für den an der Festphase gebundenen Partner als auch Bindestellen für eine der nachzuweisenden Substanzen haben. Als Rezeptoren $R_1$ können vollständige Antikörper aller Subklassen, die mit einer der nachzuweisenden Substanzen bindefähig sind, verwendet werden. In diesem Fall werden an der Oberfläche der Festphase entweder gegen den Fc-Teil des für $R_1$ verwendeten Antikörpers gerichtete Antikörper oder Protein A gebunden. Im letzteren Fall sollte der markierte Rezeptor kein vollständiger Antikörper sein, um keine unspezifische Bindung an der Festphase zu verursachen, die zu einer Verfälschung des Ergebnisses führen würde. In einer weiteren Ausführungsform werden als Rezeptoren $R_1$ Konjugate aus zu dem an der Festphase gebundenen Partner P1 komplementären Partnern P2 sowie einem mit einer der nachzuweisenden Substanzen bindefähigen Liganden eingesetzt. Über den Partner P2, der mit Partner P1 bindet, wird die Immobilisierung bewirkt. Bevorzugt enthält Rezeptor $R_1$ als Partner P1 ein Hapten wie Biotin. Der Ligand bindet mit einer der nachzuweisenden Substanzen. Abhängig von der nachzuweisenden Substanz kann der Ligand ein Antikörper, Antikörperfragment, Antigen, Hapten oder Bindeprotein sein. Als Antigene können vollständige oder gereinigte Teile von Krankheitserregern wie bakterielle Antigene, Protozoen-Antigene, Allergene oder virale Antigene, verwendet werden. Die Antigene können aus nativem Material, rekombinantem Material oder aus chemisch synthetisierten oder modifizierten Peptiden oder Kohlenhydraten bestehen. So werden beispielsweise als Liganden für die Rezeptoren $R_1$

beim Nachweis von HIV-Antikörpern verschiedene antigene Determinanten wie p24, gp41 und gp32 verwendet. Soll beispielsweise Art und Verlauf einer Hepatitis-Erkrankung nachgewiesen werden, so können für Rezeptor $R_1$ als Liganden die verschiedenen Virus-Antigene HBcAg, HBsAg, HBeAg, HAV etc. verwendet werden. Wird das Verfahren zum Nachweis von Allergien eingesetzt, so werden als Liganden die verschiedenen Allergene verwendet, um das Vorliegen von entsprechenden Antikörpern nachzuweisen. Für den Nachweis von Tumormarkern werden die entsprechenden verschiedenen Antikörper für Rezeptor $R_1$ eingesetzt, beispielsweise können Antikörper gegen CEA, AFP, CA15.3 nebeneinander für die Rezeptoren $R_1$ verwendet werden. Zur Hormonbestimmung kommen Antikörper gegen TSH, LH, FSH, Cortisol, Prolactin etc. in Frage. Ebenso können zum Nachweis von Krankheiten Antikörper gegen verschiedene virale, bakterielle oder Protozoen-Antigene eingesetzt werden. Statt der Antikörper können selbstverständlich auch deren Fragmente wie Fab-, Fab'- oder $F(ab')_2$-Fragmente verwendet werden. Die Antikörper können polyklonal oder monoklonal sein.

Der zweite, bei dem erfindungsgemäßen Verfahren verwendete Rezeptor $R_2$ ist ein Konjugat aus einem mit einer der zu bestimmenden Substanzen bindefähigen Liganden und einer Markierung. Zur Markierung sind viele Möglichkeiten bekannt und hier im vorliegenden Verfahren geeignet. Als Beispiele können radioaktive Isotope wie $^{125}J$, $^{131}J$, $^{51}Cr$, $^{35}S$ und $^3H$, Enzyme wie Peroxidase, $\beta$-Galactosidase oder alkalische Phosphatase, fluoreszierende, chemilumineszierende oder in anderer Weise nachweisbare Substanzen verwendet werden. Der für $R_2$ verwendete Ligand kann entweder eine mit allen nachzuweisenden Substanzen bindefähige Komponente sein, z.B. in dem Fall, in dem verschiedene Antigene zum Nachweis ganz bestimmter Antikörper, wie z.B. HIV, verwendet werden. In diesem Fall ist auch die Markierung für alle Rezeptoren $R_2$ gleich. Analoges gilt für die Simultanbestimmung von IgE-Antikörper verschiedener Allergene. Wenn verschiedene Parameter nebeneinander bestimmt werden sollen, können für die Rezeptoren $R_2$ jeweils verschiedene, mit den einzelnen nachzuweisenden Substanzen spezifisch bindefähige Liganden verwendet werden. Die Bestimmung der einzelnen Parameter nebeneinander kann in zwei Varianten erfolgen. In einer ersten Variante wird das Verfahren mehrphasig durchgeführt. Hier werden die verschiedenen Rezeptoren $R_2$, die jeweils die gleiche Markierung tragen, nacheinander zugegeben. Da jeder Rezeptor $R_2$ jeweils nur mit einer nachzuweisenden Substanz reagieren kann, kann nach Zugabe des Rezeptors jeweils der Anteil an gebundener oder ungebundener Markierung bestimmt werden und ist ein Maß für die jeweilige nachzuweisende Substanz. In einer zweiten Variante wird das Verfahren einphasig durchgeführt. Diese Variante ist bevorzugt, da sie auch in Analyseautomaten durchgeführt werden kann. Hier tragen die verschiedenen Rezeptoren $R_2$ jeweils verschiedene Markierungen, die eine gleichzeitige Bestimmung ermöglichen, z.B. verschiedene Isotope mit unterscheidbaren Emissionen oder auf verschiedenen Wellenlängen fluoreszierende Verbindungen. Ebenso geeignet ist die Markierung mit verschiedenen Enzymen. Hier werden zur Auswertung verschiedene Substrate zugegeben, die bei der Einwirkung der jeweiligen Enzyme Farben bilden, die ihr Absorptionsmaximum bei unterschiedlichen Wellenlängen haben. Als Liganden für Rezeptor $R_2$ sind die gleichen Liganden wie für Rezeptor $R_1$ geeignet. Die für Rezeptor $R_2$ verwendeten Liganden können abhängig von der durchzuführenden Bestimmung identisch mit den für Rezeptor $R_1$ verwendeten Liganden sein oder davon verschieden. Für den Nachweis von Antigenen können als Liganden gegen die Antigene gerichtete Antikörper oder deren Fragmente sowie Bindeproteine verwendet werden.

Für den Nachweis von Antikörpern können z.B. gegen IgG, IgA, IgM, IgE gerichtete Antikörper oder deren Fragmente oder die mit den nachzuweisenden Antikörpern bindefähigen Antigene oder Haptene eingesetzt werden. Die Rezeptoren $R_2$ können in Form von Mischungen oder in Form vernetzter Produkte eingesetzt werden. Eine Vernetzung kann in an sich bekannter Weise erfolgen, beispielsweise durch Einsatz von bi- oder polyfunktionellen Linkern. Verfahren hierzu sind dem Fachmann bekannt und bedürfen keiner näheren Erläuterung.

Die Probelösung kann entweder mit allen Rezeptoren gleichzeitig oder zuerst mit Rezeptor $R_1$ und dann mit den verschiedenen Rezeptoren $R_2$ inkubiert werden. Weitere Verfahrensvarianten sind möglich und dem Fachmann bekannt. Die Reaktion mit den beiden Rezeptoren $R_1$ und $R_2$ erfolgt dabei in homogener Phase.

Mit dem erfindungsgemäßen Verfahren gelingt es, Simultanbestimmungen für viele verschiedene Parameter durchzuführen. Die einfache Verfahrensführung ermöglicht die Simultanbestimmung in Analysenautomaten.

Durch die erfindungsgemäße Verwendung einer mit einem Partner eines spezifisch bindefähigen Paares beschichteten Oberfläche steht genug Bindungskapazität zur Verfügung, so daß alle Parameter genau und reproduzierbar nachgewiesen werden können.

Das Verfahren eignet sich auch für die Schnelldiagnose. Durch simultanen Nachweis von HIV-Antikörpern und HBsAg können Blutproben, die diese Risikofaktoren enthalten, sofort ausgeschieden werden, ohne daß eine genauere Diagnostik notwendig wäre. Andererseits kann bei Untersuchungen in einem Schnelltest vorweg geprüft werden, ob Risikofaktoren vorliegen und falls dies der Fall ist, dann eine genauere Diagnose angeschlossen werden. Es ist somit erfindungsgemäß möglich, sehr schnell festzustellen, ob bestimmte Hinweise auf eine Erkrankung vorhanden sind, die dann die weiteren Untersuchungen vereinfachen.

Die Erfindung wird durch die folgenden Figuren und Beispiele erläutert.

Fig. 1     zeigt ein Schema für eine simultane Bestimmung von HIV-Antikörpern und HBsAg.

Fig. 2     zeigt ein Schema für eine Simultanbestimmung verschiedener Tumormarker.

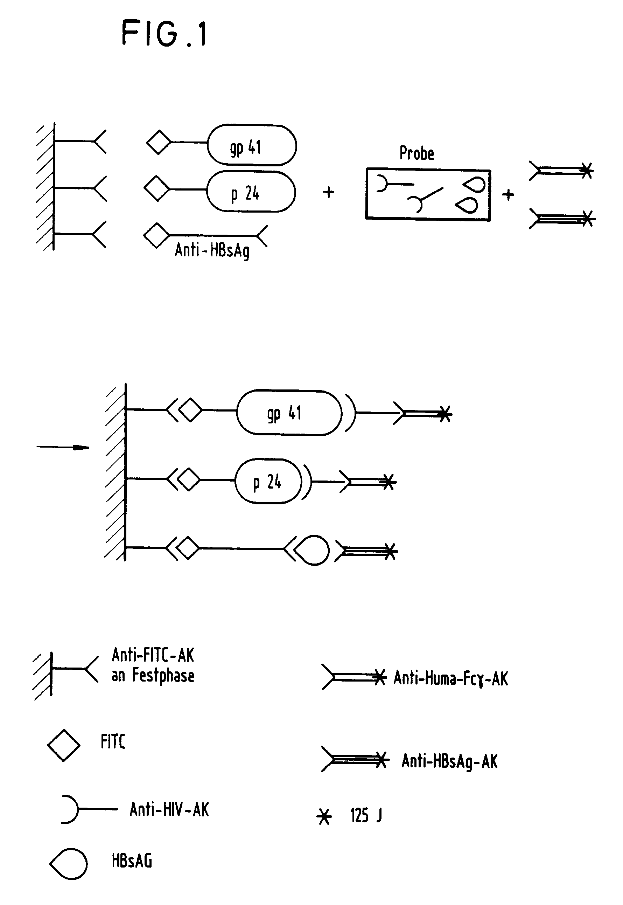

In Fig. 1 ist schematisch die simultane Bestimmung von HIV-Antikörpern und HBsAg dargestellt. An einer Festphase sind Antikörper, die gegen Fluoresceinisothiocyanat (FITC) gerichtet sind, immobilisiert. Die Probelösung wird inkubiert mit verschiedenen Konjugaten als Rezeptoren $R_1$, die alle FITC und jeweils mindestens ein mit HIV-Antikörpern bindefähiges Epitop bzw. Anti-HBsAg-Antikörper enthalten. Nach einem Waschschritt wird die Probe inkubiert mit markierten Anti-Human-IgG-Antikörpern und Anti-HBsAg-Antikörpern als Rezeptoren $R_2$. Die Markierung ist radioaktives Jod. Es bilden sich dann Komplexe aus den Rezeptoren $R_1$, HIV-Antikörpern bzw. HBsAg und aus Rezeptoren $R_2$, die durch die Bindung von FITC an die immobilisierten Anti-FITC-Antikörper an der Festphase gebunden werden.

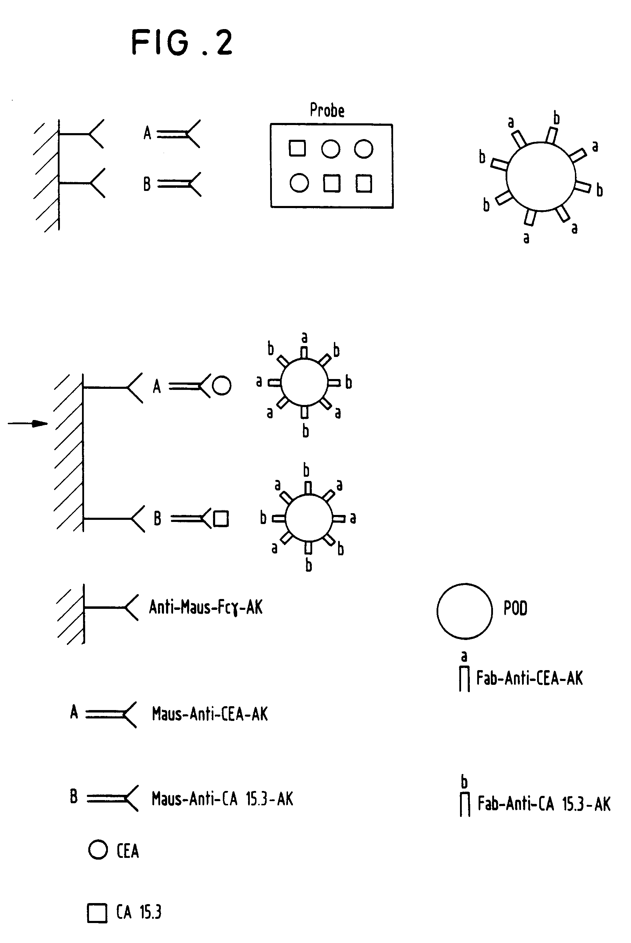

Fig. 2 zeigt eine schematische Darstellung einer simultanen Bestimmung von Tumor-Antigenen. Hier wird an der Festphase Anti-Fc$\gamma$-Antikörper immobilisiert. Als Rezeptoren $R_1$ werden jeweils gegen Tumor-antigene gerichtete Antikörper verwendet. Als Rezeptor $R_2$ wird vernetzte Peroxidase, die Fab-Fragmente von gegen Tumorantigene gerichteten Antikörpern trägt, verwendet. Bei der Inkubation binden die Anti-Tumor-Antigen-Antikörper die in der Probe vorhandenen Tumor-Antigene. Weiterhin binden die vernetzten POD-Moleküle über die entsprechenden Fab-Antikörper ebenfalls an die Tumor-Antigene. Die Immobilisierung erfolgt durch Bindung über die Anti-Fc$\gamma$-Antikörper.

**Beispiel 1**

Anti-HIV-Test

HIV-Antikörper werden in einem 2-Schritt-Sandwich-Immunoassay bestimmt. Zum Nachweis werden die Reagenzien mit folgender Zusammensetzung verwendet.

Reagenz 1:

jeweils $10^{-7}$ mol/l eines oder mehrerer biotinylierter HIV-Antigene,
40 mmol/l Phosphatpuffer, pH 7,0
0,9 Gew.-% Kochsalz
10 Vol.-% Rinderserum.

Als Antigene wurden dabei folgende verwendet: gentechnologisch hergestellte HIV-Antigene entsprechend HIV1-gp41 (gp41-rek., Centocor™-p121) und HIV1-p24 (p24-rek., Centocor™-pg2), chemisch synthetisierte Peptide aus HIV1-gp41 (gp41-pep, Wand et al., PNAS, <u>83</u>, 6159, 1986) und HIV2-gp32 (gp32-pep, Gnann, J.W. et al., Science, <u>237</u>, 1346, 1987). Diese Antigene wurden wie von Leary et al., PNAS, <u>80</u>, 4045 (1983) beschrieben mit Biotin markiert.

Reagenz 2:

20 mU/ml eines Konjugates aus Schafantikörper gegen Human-Immunglobulin und POD
40 mmol/l Phosphatpuffer, pH 7,0
0,05 Gew.-% Tween 20
0,2 % Rinderserumalbumin
0,2 % Rinder-IgG.

Polystyroltube

Thermisch aggregiertes RSA, im folgenden als Thermo-RSA bezeichnet, wurde in folgender Weise hergestellt: 1 g RSA wurde in 100 ml 50 mmol Kaliumphosphatlösung bei einem pH von 7,0 gelöst, auf 70 °C erhitzt und 4 Stunden unter leichtem Rühren bei dieser Temperatur gehalten. Die Lösung wurde abgekühlt, filtriert und auf eine Konzentration von 50 mg/ml eingestellt. Anschließend wurde gegen das 30fache Volumen bidest. Wasser dialysiert.

Herstellung eines Konjugats von Streptavidin mit Thermo-RSA: Aus Streptomyces avidinii gewonnenes Streptavidin wurde mit Maleimido-hexanoyl-N-hydroxy-succinimid umgesetzt, und man erhielt auf diese Weise ein Maleimidogruppen tragendes Streptavidin. Thermo-RSA wurde mit S-Acetylmercaptobernsteinsäureanhydrid umgesetzt und anschließend die geschützten SH-Gruppen durch Zugabe von Hydroxylamin freigesetzt. Das Maleimidogruppen enthaltende Streptavidin wurde sodann mit dem SH-Gruppen enthaltenden Thermo-RSA vermischt unter Bildung des gewünschten Konjugats.

Kunststofftubes aus Polystyrol wurden dann mit dem Streptavidin-Thermo-RSA-Konjugat beladen. Das Beladen der Tubes erfolgte mit 1,5 ml einer Streptavidin-Thermo-RSA-Lösung, wobei das molare Verhältnis der beiden Komponenten 1,8:1 (10 $\mu$g/ml) in 40 mmol/l Natriumphosphatpuffer, pH 7,4 bei 20°C während 18 bis 24 Stunden betrug.

Nach Aussaugen der Tubes wurde 30 Minuten bei 20°C mit 1,8 ml einer Lösung von 2 % Saccharose, 0,9 % Natriumchlorid und 0,3 % RSA nachbeladen. Nach Trocknung (24 Stunden bei 20°C und 40 % relativer Feuchte) waren die Tubes einsatzbereit und haltbar.

In einem mit Streptavidin-Thermo-RSA-Konjugat beschichteten Polystyroltube wurden 1 ml des Reagenz 1 und 10 $\mu$l Humanserum oder -plasma eine Stunde bei Raumtemperatur inkubiert. Anschließend wurde dreimal mit Leitungswasser gewaschen und 1 ml Reagenz 2 für eine Stunde bei Raumtemperatur inkubiert. Wieder wurde dreimal mit Leitungswasser gewaschen und zur Nachweisreaktion 1 ml ABTS-Substratlösung zugegeben. Nach 60 Minuten wurde die Extinktion bei 422 nm photometrisch gemessen.

Der Anti-HIV-Test wurde unter Verwendung einzelner HIV-Antigene und Antigenkombinationen durchgeführt. Dabei zeigte es sich, daß die Testführung im mit Streptavidin-Thermo-RSA-Konjugat beschichteten Polystyroltube für die simultane Bestimmung von mehreren Antikörpern oder Antikörperpopulationen geeignet ist (Screeningtest; Tabelle 1), gleichgültig, ob diese gegen denselben Virus oder mehrere Viren bzw. interessierende Antigene gerichtet sind.

## T A B E L L E   1

Humanserum-Probe

| HIV-Antigene | Neg. | Anti-HIV I | Anti-HIV I | Anti-HIV I | Anti-HIV II | Anti-HIV I | Anti-HIV II |
|---|---|---|---|---|---|---|---|
| | | | | Extinktion (mE) | | | |
| gp41-rek. | 52 | 1250 | 812 | 521 | 102 | 223 | 75 |
| p24-rek. | 43 | 786 | 1515 | 212 | 491 | 1531 | 263 |
| gp41-pep | 71 | 831 | 301 | 295 | 63 | 52 | 59 |
| gp32-pep | 38 | 41 | 50 | 62 | 1819 | 45 | 810 |
| gp41-, p24-rek. | 45 | 1402 | 1818 | 618 | 550 | 1559 | 266 |
| gp41-, p24-rek. + gp32-pep | 55 | 1380 | 1753 | 599 | 1723 | 1529 | 878 |

**Beispiel 2**

In einer Probelösung wurden Hepatitis S-Antigen und HIV-Antikörper in einem 2-Schritt-Sandwich-Immunoassay bestimmt. Die verwendeten Reagenzien hatten die folgende Zusammensetzung:

Reagenz 1:

40 mmol/l Phosphatpuffer, ph 7,0
0,1 % Tween 20
0,9 % NaCl
0,01 % Phenol
0,2 % Rinderserumalbumin
300 ng/ml eines FITC-markierten monoklonalen Antikörpers gegen HBSAg sowie je 600 ng/ml FITC-markiertes p24 und gp41-Antigen von HIV.
(FITC = Fluoresceinisothiocyanat)

Reagenz 2:

40 mmol/l Phosphatpuffer, pH 7,0
0,1 % Tween 20
0,2 mol/l Natriumtartrat
0,01 % Phenol
0,2 % Rinderserumalbumin
je 5000 cpm eines $^{125}$J markierten polyklonalen Schafantikörper gegen humane IgG-Antikörper sowie eines monoklonalen Antikörpers gegen HBSAg.

Polystyrolröhrchen wurden mit 1 ml Schafantikörper gegen FITC (50 $\mu$g/ml in 0,1 mol/l Carbonatpuffer, pH 9,6) für 18 Stunden bei Raumtemperatur und mit 1 ml Rinderserumalbumin (10 mg/ml in 0,1 mol/l Phosphatpuffer) für eine Stunde bei Raumtemperatur beschichtet.

50 $\mu$l Probe wurden für 2 Stunden bei Raumtemperatur mit 1 ml Reagenz 1 inkubiert. Nach dreimaligem Waschen mit Wasser wurde für eine Stunde bei Raumtemperatur mit 1 ml Reagenz 2 inkubiert. Nach dreimaligem Waschen wurden die Röhrchen im Gammazähler vermessen. Die Ergebnisse sind in Tabelle 2 dargestellt.

Tabelle 2

| Probe | HBSAg (ng/ml) | Anti-HIV (Westernblott) | Meßwert im Simultantest |
|-------|---------------|-------------------------|-------------------------|
| 1 | 12 | positiv | 2125 cpM |
| 2 | <0,2 | negativ | 52 cpM |
| 3 | <0,2 | positiv | 1159 cpM |
| 4 | <0,2 | positiv | 3041 cpM |
| 5 | <0,2 | negativ | 47 cpM |
| 6 | 125 | negativ | 830 cpM |
| 7 | 203 | negativ | 1102 cpM |
| 8 | <0,2 | negativ | 69 cpM |
| 9 | <0,2 | negativ | 29 cpM |
| 10 | <0,2 | negativ | 58 cpM |
| 11 | <0,2 | positiv | 2552 cpM |
| 12 | <0,2 | negativ | 54 cpM |
| 13 | 52 | negativ | 472 cpM |
| 14 | <0,2 | negativ | 39 cpM |
| 15 | <0,2 | negativ | 43 cpM |

**Beispiel 3**

In einer Probelösung wurden gleichzeitig die Tumormarker CEA und CA15.3 in einem 1-Schritt-Sandwich-Immunoassay bestimmt. Das verwendete Reagenz hatte die folgende Zusammensetzung:
120 mU/ml eines Konjugats aus POD und Fab-Fragmenten jeweils eines monoklonalen Antikörpers gegen die Tumormarker CEA und CA15.3
40 mmol/l Phosphatpuffer, pH 7,0
0,5 % Pluronic F68
0,2 mol/l Natriumtartrat

0,01 % Phenol

0,2 % Rinderserumalbumin

300 ng/ml jeweils eines biotinylierten monoklonalen Antikörpers gegen CEA und CA15.3.

An die monoklonalen Antikörper gegen CEA und CA15.3 werden lediglich die Anforderungen gestellt, daß die Fab-Fragmente im POD-Konjugat jeweils gegen ein anderes Epitop gerichtet sind als die biotinylierten Antikörper.

Polystyrolröhrchen wurden mit 1 ml Schaf-Anti-Maus-Fc$_\gamma$-Antikörper (50 $\mu$g/ml in 0,1 mol/l Carbonatpuffer, pH 9,6) für 18 Stunden bei Raumtemperatur und mit 1 ml Rinderserumalbumin (10 mg/ml in 0,1 mol/l Phosphatpuffer) für eine Stunde bei Raumtemperatur beschichtet. 1 ml des Reagenzes und 100 $\mu$l Probe wurden 2 Stunden bei Raumtemperatur inkubiert. Anschließend wurde dreimal mit Leitungswasser gewaschen. Sodann wurde zur Nachweisreaktion 1 ml ABTS®-Substratlösung zugegeben. Nach einer Stunde wurde die Extinktion bei 405 nm photometrisch gemessen. Die Ergebnisse sind Tabelle 3 zu entnehmen.

Tabelle 3

| Simultanbestimmung von Tumormarkern | | | |
|---|---|---|---|
| Probe | Gehalt | | Meßwert im Simultantest |
| | CEA (ng/ml) | CA15.3 (U/ml)1 | |
| 1 | <0,5 | 4 | 76 mE |
| 2 | 453 | 6 | 3580 mE |
| 3 | 2 | 5 | 58 mE |
| 4 | 212 | 7 | 2950 mE |
| 5 | 4 | 5 | 76 mE |
| 6 | 9 | 75 | 43 mE |
| 7 | 5 | 3 | 793 mE |
| 8 | 6 | 4 | 72 mE |
| 9 | 50 | 9 | 250 mE |
| 10 | 8 | 7 | 83 mE |
| 11 | 3 | 192 | 59 mE |
| 12 | 9 | 4 | 1483 mE |
| 13 | 3 | 4 | 63 mE |
| 14 | 5 | | 51 mE |

**Patentansprüche**

1. Verfahren zur Bestimmung von zwei oder mehr immunologisch nachweisbaren Substanzen nach dem Prinzip des heterogenen Immunoassays, bei dem man über eines der Bindepaare Biotin/Avidin oder Biotin/Streptavidin mehrere verschiedene Rezeptoren R$_1$, welche Bindestellen für eine der zu bestimmenden Substanzen aufweisen, an eine feste Phase koppelt, wobei die Kopplung so erfolgt, daß einer der beiden Partner des Bindesystems an ein lösliches Protein mit einem Molekulargewicht über 500000 gebunden wird, das dann an der festen Phase adsorbiert wird, und der andere Partner des Bindesystems an die Rezeptoren R$_1$ gebunden wird, die feste Phase mit den daran gebundeen Rezeptoren R$_1$ mit einer Probelösung in Kontakt bringt, welche die zu bestimmenden Substanzen enthält, und mit Hilfe von Rezeptoren R$_2$, welche Konjugate aus einem mit einer zu bestimmenden Substanz bindefähigen Liganden und einer Markierung sind, die An- oder Abwesenheit der Substanzen nachweist.

2. Verfahren nach Anspruch 1 , **dadurch gekennzeichnet,** daß für Rezeptor R$_2$ monoklonale Antikörper verwendet werden.

3. Verfahren nach einem der vorhergehenden Ansprüche, **dadurch gekennzeichnet,** daß in einer Probelösung gleichzeitig verschiedene Antikörper nachgewiesen werden.

4. Verfahren nach einem der Ansprüche 1 bis 3, **dadurch gekennzeichnet,** daß in einer Probelösung gleichzeitig verschiedene Tumormarker, Hormone, Allergene oder Krankheitserreger nachgewiesen werden.

**5.** Verfahren nach einem der vorhergehenden Ansprüche, **dadurch gekennzeichnet,** daß man als feste Phase ein Reaktionsgefäß verwendet, dessen Wände an der Innenoberfläche mit Streptavidin oder Avidin beschichtet sind.

**6.** Reagenz zur Bestimmung von immunologisch nachweisbaren Substanzen, umfassend eine feste Phase, an deren Oberfläche ein Konjugat aus einem löslichen Protein mit einem Molekulargewicht über 500.000 und einem Partner der Bindepaare Biotin/Avidin und Biotin/Streptavidin gebunden ist, verschiedene Rezeptoren $R_1$, die Konjugate aus dem komplementären Partner zu dem an der Festphase gebundenen Partner des jeweiligen Bindepaares und einem mit einer der nachzuweisenden Substanzen bindefähigen Liganden sind und Rezeptoren $R_2$, die Konjugate aus einem mit einer zu bestimmenden Substanz bindefähigen Liganden und einer Markierung sind.

**7.** Reagenz nach Anspruch 6, **dadurch gekennzeichnet,** daß die Festphase ein innen mit dem Streptavidin- oder Avidinkonjugat beschichtetes Reaktionsgefäß, die Rezeptoren jeweils Konjugate aus Biotin und jeweils einem mit einer zu bestimmenden Substanz bindefähigen Liganden sind.

**Claims**

**1.** Method for the determination of two or more immunologically detectable substances according to the heterogeneous immunoassay principle wherein several different receptors $R_1$ which have binding sites for one of the substances to be determined are coupled via one of the binding pairs biotin/avidin or biotin/streptavidin to a solid phase in which the coupling is carried out such that one of the two partners of the binding system is bound to a soluble protein with a molecular weight over 500000 which is then adsorbed to the solid phase and the other partner of the binding system is bound to the receptors $R_1$, the solid phase with the receptors $R_1$ bound thereto is brought into contact with a sample solution which contains the substances to be determined and the presence or absence of the substances is detected with the aid of receptors $R_2$ which are conjugates of a ligand capable of binding to a substance to be determined and a label.

**2.** Method as claimed in claim 1,
**wherein**
monoclonal antibodies are used for receptor $R_2$.

**3.** Method as claimed in one of the previous claims,
**wherein**
different antibodies are detected simultaneously in a sample solution.

**4.** Method as claimed in one of the claims 1 to 3,
**wherein**
different tumour markers, hormones, allergens or pathogens are detected simultaneously in a sample solution.

**5.** Method as claimed in one of the previous claims,
**wherein**
a reaction vessel is used as the solid phase whose walls are coated on the inner surface with streptavidin or avidin.

**6.** Reagent for the determination of immunologically detectable substances comprising a solid phase on whose surface a conjugate of a soluble protein with a molecular weight over 500000 and one partner of the binding pairs biotin/avidin and biotin/streptavidin is bound, different receptors $R_1$ which are conjugates of the partner of the particular binding pair that is complementary to the partner bound to the solid phase and of a ligand capable of binding to one of the substances to be detected and receptors $R_2$ which are conjugates of a ligand capable of binding to a substance to be detected and a label.

**7.** Reagent as claimed in claim 6,
**wherein**
the solid phase is a reaction vessel coated on the inside with streptavidin or avidin, the receptors are each conjugates of biotin and, in each case, one ligand capable of specific binding to a substance to be determined.

**Revendications**

**1.** Procédé pour déterminer deux ou plus de deux substances qui peuvent être mises en évidence immunologiquement selon le principe de l'immunoanalyse hétérogène, dans lequel, par l'intermédiaire de l'une des paires de liaison biotine/avidine ou biotine/streptavidine, on couple à une phase solide plusieurs récepteurs $R_1$ différents qui présentent des sites de liaison pour l'une des substances à déterminer, le couplage se faisant de telle manière que l'un des deux partenaires du système de liaison est lié à une protéine soluble de masse moléculaire supérieure à 500000 qui est ensuite adsorbée sur la phase solide, et l'autre partenaire du système de liaison est lié aux récepteurs $R_1$, on met en contact la phase solide et les récepteurs $R_1$ qui sont liés à cette phase avec une solution échantillon qui contient les substances à déterminer, et à l'aide de récepteurs $R_2$ qui sont des conjugués constitués par un ligand capable de se lier à une substance à déterminer et par un marqueur, on met en évidence la présence ou l'absence des substances.

**2.** Procédé selon la revendication 1, caractérisé en ce que l'on utilise des anticorps monoclonaux pour le récepteur $R_2$.

**3.** Procédé selon l'une des revendications précédentes, caractérisé en ce que différents anticorps sont mis en évidence simultanément dans une solution échantillon.

**4.** Procédé selon l'une des revendications 1 à 3, caractérisé en ce que différents marqueurs tumoraux, différentes hormones, différents allergènes ou différents agents pathogènes sont mis en évidence simultanément dans une solution échantillon.

**5.** Procédé selon l'une des revendications précédentes, caractérisé en ce que l'on utilise comme phase solide un récipient réactionnel dont les parois sont recouvertes sur la surface interne de streptavidine ou d'avidine.

**6.** Réactif pour déterminer des substances qui peuvent être mises en évidence immunologiquement, comportant une phase solide sur la surface de laquelle est lié un conjugué constitué par une protéine soluble d'une masse moléculaire supérieure à 500000 et par un partenaire des paires de liaison biotine/avidine et biotine/streptavidine, différents récepteurs $R_1$ qui sont des conjugués constitués par le partenaire complémentaire du partenaire de la paire de liaison correspondante qui est lié à la phase solide et par un ligand capable de se lier à l'une des substances à déterminer et des récepteurs $R_2$ qui sont des conjugués constitués par un ligand capable de se lier à l'une des substances à déterminer et par un marqueur.

**7.** Réactif selon la revendication 6, caractérisé en ce que la phase solide est un récipient réactionnel recouvert à l'intérieur de conjugué de streptavidine ou d'avidine, les récepteurs étant dans chaque cas des conjugués constitués par la biotine et par un ligand capable de se lier à l'une des substances à déterminer.

# FIG.1

# FIG .2

Probe

Anti-Maus-Fcγ-AK

POD

A === Maus-Anti-CEA-AK

a
∏ Fab-Anti-CEA-AK

B === Maus-Anti-CA 15.3-AK

b
∏ Fab-Anti-CA 15.3-AK

○ CEA

□ CA 15.3